# EUROPEAN PATENT APPLICATION

(11) **EP 3 372 245 A1**
(43) Date of publication of application: **12.09.2018**
(21) Application number: 16862084.7
(22) Date of filing: 01.11.2016
(51) Int. Cl.: A61K 47/12, A61K 9/28, A61K 47/10

(54) **METHOD FOR ACCELERATING SUGAR COATING FORMATION FOR FORMING SUGAR COATING COMPOSED OF SUGAR ALCOHOL WITH CALCIUM LACTATE**

(30) Priority: 02.11.2015 JP 2015215371
(71) Applicant: Mitsubishi Shoji Foodtech Co., Ltd., Chiyoda-ku Tokyo 100-0006 (JP)
(72) Inventor: OKI, Shiho, Fuji-shi Shizuoka 417-0033 (JP); ISHIGURO, Jun, Fuji-shi Shizuoka 417-0033 (JP)
(74) Representative: Casalonga
(86) International application number: PCT/JP2016/082473
(87) International publication number: WO 2017/078024

(57) **Abstract**

[Problem]

To provide a method for forming a smooth sugar-coating layer having sufficient practical strength that enables to significantly shorten the time required for the sugar-coating process with sugar alcohol by increasing the crystallization rate of sugar alcohol such as maltitol, etc., as well as a sugar-coating formation accelerating agent.

[Solution]

A method for accelerating the sugar-coating formation of sugar alcohol coating by blending calcium lactate in a ratio of 1 to 10% by weight in the sugar-coating liquid composed of a sugar alcohol, wherein the calcium lactate acts as accelerating agent for the sugar-coating formation of the sugar alcohol.

## Description

### Technical field

The present invention relates to shortening the sugar-coating time for sugar alcohol coating with calcium lactate. More particularly, the present invention relates to shortening the sugar-coating formation time based on the acceleration of sugar alcohol crystallization with calcium lactate.

### Prior art

Among sugar-coating processes with a sugar alcohol, sugar-coating with maltitol is one of the best-known sugar-less coating method, and is widely adopted instead of sugar-coating with sucrose due to its excellent moisture absorption stability. Maltitol is a sugar alcohol obtained by hydrogenation of maltose. Because maltitol has half of the calories of sucrose and 80 to 90% of its sweetness, and because its quality of taste is similar to that of sucrose, it is widely used as low-calorie sweetener forming an alternative to sucrose.

However, as the time for crystallization from the supersaturated solution is longer for maltitol than for sucrose, the process for crystallization from the sugar-coating liquid when performing hard sugar-coating with maltitol is time consuming, causing the completion of sugar-coated tablets to take time. This is why attempts have been made in the past to shorten the time of the sugar-coating process.

For example, JPH07-132051(A) discloses an improved hard coating process enabling easy and speedy formation of a hard coating (paragraph 0001). Concretely, while this method solves the problem by combining a polyol powder of very high purity with a crystallizable syrup containing a majority of the same polyol in relation to its soluble dry material during each cycle (paragraph 0032), it presents the drawbacks of having to incorporate a process of adding the powder in each cycle, and producing sugar-coated tablets which surface lacks of smoothness, tends to absorb moisture and does not have a crunchy feel.

### Prior art documents

### Patent document

Patent document 1: JPH07-132051(A)

### Summary of the invention

### Problem to be solved by the invention

The present invention aims at shortening the time required for the sugar-coating formation for hard sugar-coating with a sugar alcohol, e.g. maltitol, as well as at forming a smooth sugar-coating layer having a sufficient practical strength.

### Means to solve the problem

As a result of earnest research to solve the above problem, the present inventors have found that adding calcium lactate as a component of the sugar alcohol coating liquid not only enables to enhance the spreadability of the sugar-coating liquid and thereby to improve the smoothness of the sugar-coating layer, but also causes a rise in the crystallization rate of the sugar alcohol, e.g. maltitol, etc., and enables to significantly shorten the time required for the sugar-coating process.

Here, while the present invention is characterized in using calcium lactate in a sugar-coating liquid composed of a sugar alcohol, there are plenty of examples using calcium lactate for ordinary sugar-coating with sucrose in the aim of improving the sugar-coating layer (for example, JPS48-37815(B1), specification of JP2759802(B2), JPH04-261118(A), etc.). However, the effects of using calcium lactate in sugar-coating with sucrose consist in producing the precipitation of fine sucrose crystals and thereby in forming a solid sugar-coating layer.

Calcium lactate is a water-soluble calcium salt. When performing the crystallization of a substance and adding the solution of another substance to the solution of this given substance, the crystallization rate generally falls as the added substance inhibits the crystallization. In fact, when measuring the time required for sugar-coating with sucrose when calcium lactate is added and when it is not added, one finds that the process in which calcium lactate is added requires about 10% more time up to the preparation of the sugar-coated tablet (see Comparative Examples 3 and 4, to be described later).

On the other hand, a defect of sugar-coating with a sugar alcohol is that it is time-consuming. This is due to the slow crystallization rate of sugar alcohols such as maltitol, etc. Conventionally, methods consisting in adding solids, such as a crystalline nucleus, etc., have been performed in order to increase the crystallization rate.

However, keen study by the present inventors has shown that, dissolving calcium lactate in a sugar-coating liquid composed of a sugar alcohol such as maltitol, etc., and performing sugar-coating, have, contrary to expected, an effect of accelerating the crystallization of the sugar alcohol and enables to shorten the sugar-coating time to approximately half of the conventional time, leading to the completion of the present invention.

Here, although there was some prior art relating to sugar-coating liquids that contain saccharides, calcium lactate and the like, (for example, JP2002-179559(A), JP2759802(B2), etc.), there was no suggestion of an effect of accelerating the crystallization of a sugar alcohol such as maltitol, etc., according to calcium lactate, neither was there a mention of the acceleration of the sugar-coating formation for sugar-coating with a sugar alcohol.

In other words, the present invention is, first, a method for accelerating sugar-coating formation of sugar alcohol coating using calcium lactate.

Second, the present invention is a method for accelerating sugar-coating formation according to the above first aspect, wherein the calcium lactate is comprised in a sugar-coating liquid.

Third, the present invention is a method for accelerating sugar-coating formation according to the above first or second aspect, wherein the content of calcium lactate is 1 to 10% by weight of the sugar-coating liquid.

Fourth, the present invention is a composition for accelerating the sugar-coating formation of sugar alcohol containing calcium lactate.

There is no restriction with regard to the form of the sugar alcohol to be used in the present invention as long as the formation of a sugar-coating layer is possible. For example, maltitol may be used in the form of a crystalline product, crystalline mixture solid product, or liquid product. However, from the perspective of smoothness and strength of the sugar-coating layer, maltitol having a purity of 90% or more, preferably 95% or more, more preferably 98% or more in terms of solid content may be advantageously used. Also, in addition to the above-mentioned maltitol, the sugar alcohol of the present invention may consist of sorbitol, xylitol, lactitol, reduced substance of isomaltulose, etc.

The calcium lactate to be used in the present invention may be in the form of a crystalline product or a liquid product. However, from the perspective of operability, it is preferable to use a crystalline product.

Also, the content of calcium lactate in the present invention is preferably 1 to 10% by weight of the sugar-coating liquid. A content of 10% by weight or less enables to maintain a low solution viscosity and to obtain good operability. On the other hand, a content of 1% by weight or more enables to obtain a sugar-coating layer having good smoothness and usable sugar-coating layer strength.

Furthermore, thickeners such as gum arabic, gelatin, pullulan, xanthan gum, hydroxymethyl cellulose, dietary fiber, etc., and modified starch may also be used in the sugar-coating liquid of the present invention.

### Effects of the invention

The present invention enables to significantly shorten the time required for the sugar-coating process of sugar-coating with sugar alcohol by increasing the crystallization rate of sugar alcohol such as maltitol, etc., as well as to form a smooth sugar-coating layer having sufficient practical strength.

### Modes to carry out the invention

The present invention will be described concretely with reference to the following Examples. The technical scope of the present invention is not, however, limited to these Examples. Also, unless otherwise specified, calcium lactate refers to the content as a pentahydrate.

The following raw materials were used in the present invention.
(1) Sugar-coating layer base material
   - Crystalline maltitol: Lesys (manufactured by Mitsubishi Shoji Foodtech, Co., Ltd.)
   - Maltitol fine powder: Lesys fine powder (manufactured by Mitsubishi Shoji Foodtech, Co., Ltd.)
   - Crystalline xylitol: Xylit (manufactured by Mitsubishi Shoji Foodtech, Co., Ltd.)
   - Xylitol fine powder: Xylit fine powder (manufactured by Mitsubishi Shoji Foodtech, Co., Ltd.)
   - Calcium lactate (pentahydrate): Calcium lactate (manufactured by Showa Kako Corporation)
   - Crystalline sucrose: Granulated sugar ME (manufactured by Dai-Nippon Meiji Sugar Co., Ltd.)
   - Sucrose fine powder: MGP icing sugar (manufactured by Tokukura Corporation)
(2) Sugar-coating layer compounding agents
   - Gum arabic: Powdered gum arabic (manufactured by Kanto Chemical Co., Inc.)
   - Oxidized starch: prepared as described below
(3) Raw materials for core material
   - Maltitol powder: Amalty MR (manufactured by Mitsubishi Shoji Foodtech, Co., Ltd.)
   - Magnesium stearate: Magnesium stearate (manufactured by Wako Pure Chemical Industries, Ltd.)

The following devices were used in the present invention.
- Tableting machine: VIRGO 0512SS2AZ (manufactured by Kikusui Seisakusho, Ltd.)
- Sugar-coating machine: No. 16D-S (manufactured by Kikusui Seisakusho, Ltd.)
- V mixer: V mixer (manufactured by Ikeda Scientific, Co., Ltd.)

### (Preparation of the oxidized starch)

Water (2 kg) was added to tapioca starch (1 kg) (manufactured by Nihon Shokuhin Kako Co., Ltd.) to form a suspension that is heated to 30°C. A 3% sodium hydroxide aqueous solution was added to this suspension to adjust the pH to 11.0, 150 mL of a sodium hypochlorite solution containing 13% effective chlorine was added, and an oxidation treatment was performed for one hour. Then, after neutralization with a hydrochloric acid, washing/dehydration/drying were performed to prepare oxidized tapioca starch. The obtained oxidized tapioca starch (30 parts by weight) was mixed with potato starch (70 parts by weight) (primary standard reagent manufactured by Kanto Chemical Co., Inc.) to obtain oxidized starch.

### (Preparation of the core material)

The core material used in the present invention was prepared as follows.

Magnesium stearate (2.5 parts by weight) was added as lubricant to Amalty MR (100 parts by weight) and tablet making was performed with a dosage form of 8 mm sugar-coating R, 180 mg unit weight, 4 punches, 40 rpm rotation frequency and 770 kgf tableting pressure, to produce the core material.

### (Preparation of the sugar-coating liquid)

Sugar-coating liquid (300 g) was prepared for one test section. Sugar-coating liquid components with a mass (g) corresponding to 3 times the mass ratios indicated in the Examples and Comparative Examples of Tables 1 to 7 were taken and mixed in a 500 mL beaker. Purified water was added and mixed into the substance that was then heated and dissolved in a water bath. Purified water was added to reach 300 g and the obtained substance was maintained at 60°C to prepare the sugar-coating liquid.

### (Sugar-coating process I, under hot air blow)

Sugar-coating under hot air blow was performed according to the following steps I-1 to I-7 (regarding steps I-2 and I-3, see the specified number of times shown in the respective Examples and Comparative Examples in Tables 1 to 3).

### (Step 1-1)

Core material (300 g) was fed into a sugar-coating pan, sugar-coating liquid (8 g) was added while the sugar-coating pan revolved at 30 rpm. Subsequently, the operation consisting of adding maltitol fine powder (1 g) (sucrose fine powder in Comparative Examples 3 and 4), leaving the sugar-coating liquid to spread under windless conditions for 4 min, then, drying the sugar-coating liquid under a 45°C hot air blow for 2 min, was repeated twice.

### (Step 1-2)

The operation consisting in adding sugar-coating liquid (7 g), leaving the sugar-coating liquid to spread under windless conditions for 4.5 min, then, drying the sugar-coating liquid under a 45°C hot air blow for 2 min, was repeated the specified number of times.

### (Step 1-3)

The operation consisting in adding sugar-coating liquid (9 g), leaving the sugar-coating liquid to spread under windless conditions for 6 min, then drying the sugar-coating liquid under a 45°C hot air blow for 2 min, was repeated the specified number of times.

### (Step 1-4)

In order to smooth the sugar-coating layer, the operation consisting in adding sugar-coating liquid (6 g), leaving the sugar-coating liquid to spread under windless conditions for 6 min, then, the heater of the ventilator being turned off, drying the sugar-coating liquid under air blow (22°C) for 6 min, was repeated twice (25 times in Comparative Example 1 only).

### (Step 1-5)

The amount of added sugar-coating liquid was changed to 6 g, 4 g, 3 g, and 1.5 g, in this order, and drying was respectively performed under windless conditions for 12 min, 10 min, 8 min, and 6 min.

### (Step 1-6)

The rotation speed of the sugar-coating pan was changed to 12 rpm, sugar-coating liquid (1 g) was added and the rotation of the sugar-coating pan was stopped for 30 sec. Thereafter, the operation consisting in rotating the sugar-coating pan once then stopping it for 5 sec was repeated 5 times, the opening was then covered with a wet paper towel and the sugar-coating pan was rotated for 8 min and 40 sec.

### (Step 1-7)

The sugar-coated tablets were transferred to another sugar-coating pan to be rotated at 30 rpm, and the operation consisting in adding carnauba wax in a quantity corresponding to 0.03% of the total weight of the sugar-coated tablets then rotating the sugar-coating pan for 30 min was repeated twice.

In step 1-2, sugar-coating was performed using a relatively small amount of sugar-coating liquid until the peripheral part of the tablets was covered with the sugar-coating layer. The amount of sugar-coating liquid was then increased in step 1-3 and sugar-coating was performed with a target sugar-coating ratio of 35%. Here, since the physical properties of the sugar-coating liquids used in the various Examples and Comparative Examples differed and the amount of sugar-coating layer formation for one operation therefore differed for each Example and Comparative Example, the number of times the operation was repeated in order to obtain a fixed sugar-coating ratio varied for each Example and Comparative Example.

### (Coating process II, under room temperature air blow)

Sugar-coating under room temperature air blow was performed according to the following steps II-1 to II-5 (regarding steps II-2 and II-3, see the specified number of times shown in the respective Examples and Comparative Examples in Tables 4 to 7).

### (Step II-1)

Core material (300 g) was fed into a sugar-coating pan, and sugar-coating liquid (4 g) was added while the sugar-coating pan revolved at 30 rpm. Subsequently, the operation consisting in adding maltitol fine powder (1 g) (or xylitol fine powder), drying the sugar-coating liquid under windless conditions for 1 min, then drying the sugar-coating liquid under a room temperature air blow (22°C) for 9 min, was repeated 5 times.

### (Step II-2)

The operation consisting in adding sugar-coating liquid (4 g), leaving the sugar-coating liquid to spread under windless conditions for 4 min, then drying the sugar-coating liquid under a room temperature air blow (22°C) for 4 min, was repeated the specified number of times.

### (Step II-3)

The operation consisting in adding sugar-coating liquid (4 g), leaving the sugar-coating liquid to spread under windless conditions for 6 min, then drying the sugar-coating liquid under a room temperature air blow (22°C) for 2 min, was repeated the specified number of times.

### (Step II-4)

The rotation speed of the sugar-coating pan was changed to 12 rpm, the amount of sugar-coating liquid to be added was set to 1.5 g, and the rotation of the sugar-coating pan was stopped for 30 sec. Thereafter, the operation consisting of rotating the sugar-coating pan once then stopping it for 5 sec was repeated 5 times, the opening was then covered with a wet paper towel and the sugar-coating pan was rotated for 8 min and 40 sec.

### (Step II-5)

The sugar-coated tablets were transferred to another sugar-coating pan to be rotated at 30 rpm, and the operation consisting in adding carnauba wax in a quantity corresponding to 0.03% of the total weight of the sugar-coated tablets then rotating the sugar-coating pan for 30 min was repeated twice.

An evaluation of the sugar-coated tablets obtained in the Examples and Comparative Examples was made based on the sugar-coating rate, sugar-coating time and 75-degree glossiness.

Here, the sugar-coating rate referred to in the present invention is the sugar-coating ratio to an uncoated tablet per minute and is calculated from the sugar-coating time and sugar-coating ratio.

The sugar-coating ratio is the percentage of the weight of the sugar-coating layer with respect to the weight of an uncoated tablet and is calculated by respectively measuring 10 uncoated tablets and 10 sugar-coated tablets 3 times each.

The sugar-coating time referred to in the present invention is the processing time required from the start of the sugar-coating process up to before the glazing process (from step I-1 to step I-6, or from step II-1 to step II-4).

The 75-degree glossiness referred to in the present invention is the value (JIS-Z8741-1983) measured at a gloss meter (Gloss Meter VG2000, manufactured by Nippon Denshoku Industries, Co. Ltd.). Measurement was made for 5 specimens and the mean value thereof was defined as measured value.

### Example 1

### (Maltitol sugar-coating using calcium lactate and gum arabic, under hot air blow)

Sugar-coating process I (under hot air blow) was performed using the sugar-coating liquid shown in Table 1, the number of times operations were repeated as specified by the sugar-coating process specified number of times in Table 1, to obtain a sugar-coated tablet (Example product 1). The sugar-coating rate, sugar-coating time and 75-degree glossiness of the obtained sugar-coated tablet are shown in Table 1.

### [Comparative Example 1]

### (Maltitol sugar-coating, step 1-4 (25 times), under hot air blow)

Sugar-coating process I (under hot air blow) was performed using the sugar-coating liquid shown in Table 1, the number of times operations were repeated as specified by the sugar-coating process specified number of times in Table 1, to obtain a sugar-coated tablet (Comparative product 1). The sugar-coating rate, sugar-coating time and 75-degree glossiness of the obtained sugar-coated tablet are shown in Table 1. For this Comparative Example only, step 1-4 was repeated 25 times. (For Examples and Comparative Examples under hot air blow other than this Comparative Example, the number of times step 1-4 was repeated was twice).

### [Comparative Example 2]

### (Maltitol sugar-coating, step 1-4 (twice), under hot air blow)

Sugar-coating process I (under hot air blow) was performed using the sugar-coating liquid shown in Table 1, the number of times operations were repeated as specified by the sugar-coating process specified number of times in Table 1, to obtain a sugar-coated tablet (Comparative product 2). The sugar-coating rate, sugar-coating time and 75-degree glossiness of the obtained sugar-coated tablet are shown in Table 1.

### [Table 1]

**Table 1**

| | | Example 1 | Comp. Ex. 1 | Comp. Ex. 2 |
|---|---|---|---|---|
| Sugar-coating liquid components | Crystalline maltitol | 62.0 | 65.0 | 65.0 |
| | Calcium lactate | 5.0 | - | - |
| | Gum arabic | 1.0 | 3.0 | 3.0 |
| Sugar-coating process specified number of times (Step 1-2) | | 12 | 19 | 18 |
| Sugar-coating process specified number of times (Step 1-3) | | 13 | 4 | 14 |
| Sugar-coating rate (Sugar-coating ratio per min) | | 0.146 | 0.117 | 0.144 |
| Sugar-coating time (min) | | 241.0 | 429.0 | 288.5 |
| 75-degree glossiness | | 12.7 | 10.4 | 8.9 |

In order to smooth the surface of the sugar-coated tablet with the maltitol sugar-coating indicated for Comparative product 1, the process (step 1-4) needed to be repeated 25 times. On the other hand, the process for smoothing the tablet surface (step 1-4) could be simplified for Example product 1 according to the present invention. And, while the sugar-coating time has been significantly shortened, Example product 1 was superior in terms of 75-degree glossiness, which forms an indicator of the surface smoothness. The sugar-coating time of Comparative product 2, which was manufactured according to the same process as Example product 1 but which did not use calcium lactate, was long, and the 75-degree glossiness was also by far inferior to that of the Example product. These facts allow to acknowledge that using calcium lactate enables to obtain a shortening in the time of sugar-coating with a sugar alcohol as well as an improvement in the glossiness.

### [Comparative Example 3]

### (Sucrose sugar-coating, under hot air blow)

Sugar-coating process I (under hot air blow) was performed using the sugar-coating liquid shown in Table 2, the number of times operations were repeated as specified by the sugar-coating process specified number of times in Table 2, to obtain a sugar-coated tablet (Comparative product 3). The sugar-coating rate, sugar-coating time and 75-degree glossiness of the obtained sugar-coated tablet are shown in Table 2.

### [Comparative Example 4]

### (Sucrose sugar-coating using calcium lactate, under hot air blow)

Sugar-coating process I (under hot air blow) was performed using the sugar-coating liquid shown in Table 2, the number of times operations were repeated as specified by the sugar-coating process specified number of times in Table 2, to obtain a sugar-coated tablet (Comparative product 4). The sugar-coating rate, sugar-coating time and 75-degree glossiness of the obtained sugar-coated tablet are shown in Table 2.

### [Table 2]

**Table 2**

| | | Comp. Ex. 3 | Comp. Ex. 4 |
|---|---|---|---|
| Sugar-coating liquid components | Crystalline sucrose | 65.0 | 62.0 |
| | Calcium lactate | - | 5.0 |
| | Gum arabic | 3.0 | 1.0 |
| Sugar-coating process specified number of times (Step 1-2) | | 10 | 15 |
| Sugar-coating process specified number of times (Step 1-3) | | 11 | 8 |
| Sugar-coating rate (Sugar-coating ratio per min) | | 0.151 | 0.139 |
| Sugar-coating time (min) | | 220.5 | 243.0 |
| 75-degree glossiness | | 9.5 | 8.8 |

Comparative Examples 3 and 4 show the impact caused by the use of calcium lactate on sucrose sugar-coating. Compared to Comparative Example 3 which did not use calcium lactate, Comparative Example 4 which used calcium lactate had a lower 75-degree glossiness which formed an indicator of the surface smoothness, and also required a longer sugar-coating time.

Conventional techniques that have improved sugar-coating with sucrose by using calcium lactate in the sucrose sugar-coating liquid, are based on the fact that the use of calcium lactate in sucrose causes the generation of fine sucrose crystals. Their technological background differs from that of the present invention which is based on the finding that the use of calcium lactate accelerates the crystallization of sugar alcohols such as maltitol, etc.

Therefore, the effects obtained when using calcium lactate in a sucrose sugar-coating liquid differ from those obtained when using it in a sugar-coating liquid composed of a sugar alcohol such as maltitol, etc. And it has become clear that they differ from the effects of the present invention which consist in shortening the required sugar-coating time and obtaining a smooth sugar-coating layer having sufficient practical strength.

### Example 2

### (Maltitol sugar-coating using 1% by weight of calcium lactate, under hot air blow)

Sugar-coating process I (under hot air blow) was performed using the sugar-coating liquid shown in Table 3, the number of times operations were repeated as specified by the sugar-coating process specified number of times in Table 3, to obtain a sugar-coated tablet (Example product 2). The sugar-coating rate, sugar-coating time and 75-degree glossiness of the obtained sugar-coated tablet are shown in Table 3.

### Example 3

### (Maltitol sugar-coating using 10% by weight of calcium lactate, under hot air blow)

Sugar-coating process I (under hot air blow) was performed using the sugar-coating liquid shown in Table 3, the number of times operations were repeated as specified by the sugar-coating process specified number of times in Table 3, to obtain a sugar-coated tablet (Example product 3). The sugar-coating rate, sugar-coating time and 75-degree glossiness of the obtained sugar-coated tablet are shown in Table 3.

### [Comparative Example 5]

### (Maltitol sugar-coating using 0.5% by weight of calcium lactate, under hot air blow)

Sugar-coating process I (under hot air blow) was performed using the sugar-coating liquid shown in Table 3, the number of times operations were repeated as specified by the sugar-coating process specified number of times in Table 3, to obtain a sugar-coated tablet (Comparative product 5). The sugar-coating rate, sugar-coating time and 75-degree glossiness of the obtained sugar-coated tablet are shown in Table 3.

### [Table 3]

**Table 3**

| | | Example 2 | Example 3 | Comp. Ex. 5 |
|---|---|---|---|---|
| Sugar-coating liquid components | Crystalline maltitol | 66.0 | 57.0 | 66.5 |
| | Calcium lactate | 1.0 | 10.0 | 0.5 |
| | Gum arabic | 1.0 | 1.0 | 1.0 |
| Sugar-coating process specified number of times (Step 1-2) | | 9 | 11 | 10 |
| Sugar-coating process specified number of times (Step 1-3) | | 14 | 14 | 9 |
| Sugar-coating rate (Sugar-coating ratio per min) | | 0.153 | 0.208 | 0.176 |
| Sugar-coating time (min) | | 232.0 | 208.0 | 201.5 |
| 75-degree glossiness | | 11.5 | 12.3 | 10.6 |

The amount of calcium lactate used in Examples 2 and 3 was respectively 1% by weight and 10 % by weight of the sugar-coating liquid and enabled to satisfactorily carry out the present invention. When the content of calcium lactate was 10% by weight, viscosity of the sugar-coating liquid was low and there was no impact on the operatibility. In the case of 1% by weight, one could acknowledge a sufficient glossiness as compared to Comparative Example 5.

### Example 4

### (Maltitol sugar-coating using calcium lactate and gum arabic, under room temperature air blow)

Sugar-coating process II (under room temperature air blow) was performed using the sugar-coating liquid shown in Table 4, the number of times operations were repeated as specified by the sugar-coating process specified number of times in Table 4, to obtain a sugar-coated tablet (Example product 4). The sugar-coating rate, sugar-coating time and 75-degree glossiness of the obtained sugar-coated tablet are shown in Table 4.

### [Comparative Example 6]

### (Common maltitol sugar-coating, under room temperature air blow)

Sugar-coating process II (under room temperature air blow) was performed using the sugar-coating liquid shown in Table 4, the number of times operations were repeated as specified by the sugar-coating process specified number of times in Table 4, to obtain a sugar-coated tablet (Comparative product 6). The sugar-coating rate, sugar-coating time and 75-degree glossiness of the obtained sugar-coated tablet are shown in Table 4.

### [Table 4]

**Table 4**

| | | Example 4 | Comp. Ex. 6 |
|---|---|---|---|
| Sugar-coating liquid components | Crystalline maltitol | 64.0 | 65.0 |
| | Calcium lactate | 1.0 | - |
| | Gum arabic | 3.0 | 3.0 |
| Sugar-coating process specified number of times (Step II-2) | | 46 | 55 |
| Sugar-coating process specified number of times (Step II-3) | | 17 | 21 |
| Sugar-coating rate (Sugar-coating ratio per min) | | 0.089 | 0.075 |
| Sugar-coating time (min) | | 561.8 | 666.7 |
| 75-degree glossiness | | 18.3 | 16.3 |

Sugar-coating under room temperature air blow requires a longer time for the formation of the sugar-coating layer than under hot air blow. Despite exhibiting a shorter sugar-coating time than that of Comparative product 6, Example product 4 made according to the present invention had a superior 75-degree glossiness, which forms an indicator of the surface smoothness. These facts allow to acknowledge that using calcium lactate enables to obtain a shortening in the time of sugar-coating with a sugar alcohol as well as an improvement in the glossiness.

### Example 5

### (Maltitol sugar-coating using calcium lactate and oxidized starch, under room temperature air blow)

Sugar-coating process II (under room temperature air blow) was performed using the sugar-coating liquid shown in Table 5, the number of times operations were repeated as specified by the sugar-coating process specified number of times in Table 5, to obtain a sugar-coated tablet (Example product 5). The sugar-coating rate, sugar-coating time and 75-degree glossiness of the obtained sugar-coated tablet are shown in Table 5.

### [Comparative Example 7]

### (Maltitol sugar-coating using oxidized starch, under room temperature air blow)

Sugar-coating process II (under room temperature air blow) was performed using the sugar-coating liquid shown in Table 5, the number of times operations were repeated as specified by the sugar-coating process specified number of times in Table 5, to obtain a sugar-coated tablet (Comparative product 7). The sugar-coating rate, sugar-coating time and 75-degree glossiness of the obtained sugar-coated tablet are shown in Table 5.

### [Table 5]

**Table 5**

| | | Example 5 | Comp. Ex. 7 |
|---|---|---|---|
| Sugar-coating liquid components | Crystalline maltitol | 64.0 | 65.0 |
| | Calcium lactate | 1.0 | - |
| | Oxidized starch | 3.0 | 3.0 |
| Sugar-coating process specified number of times (Step II-2) | | 46 | 55 |
| Sugar-coating process specified number of times (Step II-3) | | 18 | 22 |
| Sugar-coating rate (Sugar-coating ratio per min) | | 0.088 | 0.074 |
| Sugar-coating time (min) | | 568.2 | 675.7 |
| 75-degree glossiness | | 18.6 | 16.6 |

Similarly to the systems using gum arabic, one could acknowledge that the use of calcium lactate also caused a shortening of the sugar-coating time as well as a glossiness improvement for systems using oxidized starch as a compounding agent of the sugar-coating layer.

### Example 6

### (Xylitol sugar-coating using calcium lactate and gum arabic, under room temperature air blow)

Sugar-coating process II (under room temperature air blow) was performed using the sugar-coating liquid shown in Table 6, the number of times operations were repeated as specified by the sugar-coating process specified number of times in Table 6, to obtain a sugar-coated tablet (Example product 6). The sugar-coating rate, sugar-coating time and 75-degree glossiness of the obtained sugar-coated tablet are shown in Table 6.

### [Comparative Example 8]

### (Xylitol sugar-coating using gum arabic, under room temperature air blow)

Sugar-coating process II (under room temperature air blow) was performed using the sugar-coating liquid shown in Table 6, the number of times operations were repeated as specified by the sugar-coating process specified number of times in Table 6, to obtain a sugar-coated tablet (Comparative product 8). The sugar-coating rate, sugar-coating time and 75-degree glossiness of the obtained sugar-coated tablet are shown in Table 6.

### [Table 6]

**Table 6**

| | | Example 6 | Comp. Ex. 8 |
|---|---|---|---|
| Sugar-coating liquid components | Crystalline xylitol | 64.0 | 65.0 |
| | Calcium lactate | 1.0 | - |
| | Gum arabic | 3.0 | 3.0 |
| Sugar-coating process specified number of times (Step II-2) | | 43 | 46 |
| Sugar-coating process specified number of times (Step II-3) | | 12 | 16 |
| Sugar-coating rate (Sugar-coating ratio per min) | | 0.100 | 0.090 |
| Sugar-coating time (min) | | 500.0 | 555.6 |
| 75-degree glossiness | | 16.0 | 14.9 |

Similarly to the systems using maltitol as base material for the sugar-coating layer, one could acknowledge that the use of calcium lactate also caused a shortening of the sugar-coating time as well as a glossiness improvement for systems using xylitol as base material for the sugar-coating layer.

### Example 7

### (Xylitol sugar-coating using calcium lactate and oxidized starch, under room temperature air blow)

Sugar-coating process II (under room temperature air blow) was performed using the sugar-coating liquid shown in Table 7, the number of times operations were repeated as specified by the sugar-coating process specified number of times in Table 7, to obtain a sugar-coated tablet (Example product 7). The sugar-coating rate, sugar-coating time and 75-degree glossiness of the obtained sugar-coated tablet are shown in Table 7.

### [Comparative Example 9]

### (Xylitol sugar-coating using oxidized starch, under room temperature air blow)

Sugar-coating process II (under room temperature air blow) was performed using the sugar-coating liquid shown in Table 7, the number of times operations were repeated as specified by the sugar-coating process specified number of times in Table 7, to obtain a sugar-coated tablet (Comparative product 9). The sugar-coating rate, sugar-coating time and 75-degree glossiness of the obtained sugar-coated tablet are shown in Table 7.

### [Table 7]

**Table 7**

| | | Example 7 | Comp. Ex. 9 |
|---|---|---|---|
| Sugar-coating liquid components | Crystalline xylitol | 64.0 | 65.0 |
| | Calcium lactate | 1.0 | - |
| | Oxidized starch | 3.0 | 3.0 |
| Sugar-coating process specified number of times (Step II-2) | | 43 | 46 |
| Sugar-coating process specified number of times (Step II-3) | | 13 | 16 |
| Sugar-coating rate (Sugar-coating ratio per min) | | 0.099 | 0.089 |
| Sugar-coating time (min) | | 505.1 | 561.8 |
| 75-degree glossiness | | 16.4 | 15.4 |

Similarly to the systems using maltitol as base material for the sugar-coating layer, one could acknowledge that the use of calcium lactate caused a shortening of the sugar-coating time as well as a glossiness improvement for systems using xylitol as base material for the sugar-coating layer, irrespectively of the type of compounding agent for the sugar-coating layer.

## Claims

1. A method for accelerating sugar-coating formation of sugar alcohol coating using calcium lactate.

2. The method for accelerating sugar-coating formation according to claim 1, wherein the calcium lactate is comprised in a sugar-coating liquid.

3. The method for accelerating sugar-coating formation according to claim 1 or 2, wherein the content of calcium lactate is 1 to 10% by weight of the sugar-coating liquid.

4. A composition for accelerating the sugar-coating formation of sugar alcohol containing calcium lactate.
